# EUROPEAN PATENT APPLICATION

(11) **EP 4 792 732 A1**
(43) Date of publication of application: **19.08.2026**
(21) Application number: 24876983.8
(22) Date of filing: 12.09.2024
(51) Int. Cl.: A61B 3/103, A61B 3/10, A61B 3/135

(54) **OPHTHALMOLOGIC DEVICE**

(30) Priority: 10.10.2023 JP 2023175018
(71) Applicant: Tomey Corporation, Nagoya-shi, Aichi 451-0051 (JP)
(72) Inventor: OKAMOTO Keiichiro, Nagoya-shi, Aichi 451-0051 (JP); NAKASHIMA Masaya, Nagoya-shi, Aichi 451-0051 (JP); Liu Linsheng, Nagoya-shi, Aichi 451-0051 (JP)
(74) Representative: BSB Patentanwälte Schütte & Engelen Partnerschaft mbB
(86) International application number: PCT/JP2024/032665
(87) International publication number: WO 2025/079384

(57) **Abstract**

To provide a technique capable of realizing a device for photographing both eyes at low cost.

An ophthalmic device is configured that includes an illumination optical system that illuminates each of anterior eye portions of both eyes to be examined, an imaging optical system including a coupling mirror, a pair of mirrors that guide reflected light from each of the illuminated anterior eye portions of both eyes to a pair of the coupling mirror, and one image sensor that images each of the anterior eye portions of both eyes based on the reflected light from the coupling mirror; and a control unit that causes the image sensor to perform imaging.

## Description

### TECHNICAL FIELD

The present invention relates to an ophthalmic device.

### BACKGROUND ART

In recent years, from a viewpoint of preventive medicine, there has been a demand for a device capable of simply screening for eye diseases in commercial facilities, drug stores, and the like. Patent Literature 1 discloses that both eyes of a face are included in a photographing range in order to improve alignment workability.

### CITATIONS LIST

### PATENT LITERATURE

Patent Literature 1: JP 2021-40793 A

### SUMMARY OF INVENTION

### TECHNICAL PROBLEMS

In a case of a configuration as in Patent Literature 1, since an entire face including both eyes is photographed at a low magnification, rough alignment is possible, but it is not suitable for precise alignment. A high magnification zoom mechanism is required for precise alignment, but in that case, both eyes cannot be photographed. Furthermore, it is necessary to provide a zoom mechanism and a drive unit for moving a measurement unit in front of left and right eyes, and there is a problem that the device becomes large-scale.

The present invention has been made in view of the above problems, and an object thereof is to provide a technique capable of realizing a device for photographing both eyes at low cost.

### SOLUTIONS TO PROBLEMS

In order to achieve the above object, the ophthalmic device includes an illumination optical system that illuminates each of anterior eye portions of both eyes to be examined, an imaging optical system including a coupling mirror, a pair of mirrors that guides reflected light from each of the illuminated anterior eye portions of both eyes to a pair of coupling mirrors, and one image sensor that images each of the anterior eye portions of both eyes based on the reflected light from the coupling mirror, and a control unit that causes the image sensor to perform imaging.

In other words, since this ophthalmic device has a configuration in which both eyes are imaged by one image sensor, the cost can be reduced as compared with a case where each eye is imaged by another image sensor. Further, since both eyes can be photographed at a time by one image sensor, it is not necessary for a subject to shift the ophthalmic device left or right for right eye photographing and left eye photographing, thereby shortening an examination time.

### BRIEF DESCRIPTION OF DRAWINGS

Figs. 1A and 1B are views illustrating an external appearance of an ophthalmic device.
Fig. 2 is a block diagram illustrating a configuration of the ophthalmic device.
Fig. 3 is a diagram illustrating the configuration of an optical system according to a first embodiment.
Figs. 4A, 4B, and 4C are schematic diagrams for describing reflection in a coupling mirror.
Fig. 5A is a view illustrating an example of a captured image before processing, and Fig. 5B is a view illustrating an example of a captured image after processing.
Fig. 6 is a diagram illustrating a configuration example of a measurement optical system.
Fig. 7A is a view for describing tracking, and Fig. 7B is a view for describing scanning.
Fig. 8 is a flowchart of measurement processing.
Fig. 9 is a diagram illustrating the configuration of an optical system according to a second embodiment.
Fig. 10A is a schematic diagram for describing the configuration of an imaging optical system according to the second embodiment, and Fig. 10B is a schematic diagram illustrating a comparative example.
Fig. 11 is a diagram illustrating the configuration of an optical system as a modification of the second embodiment.
Fig. 12 is a diagram illustrating the configuration of an optical system according to a third embodiment.
Fig. 13 is a view illustrating the external appearance of an ophthalmic device according to another embodiment.
Fig. 14 is a diagram illustrating a configuration example of a measurement optical system according to another embodiment.

### DESCRIPTION OF EMBODIMENTS

Here, embodiments of the present invention will be described in the following order.
(1) First embodiment:
(2) Second embodiment:
(3) Third embodiment:
(4) Other embodiments:

### (1) First embodiment:

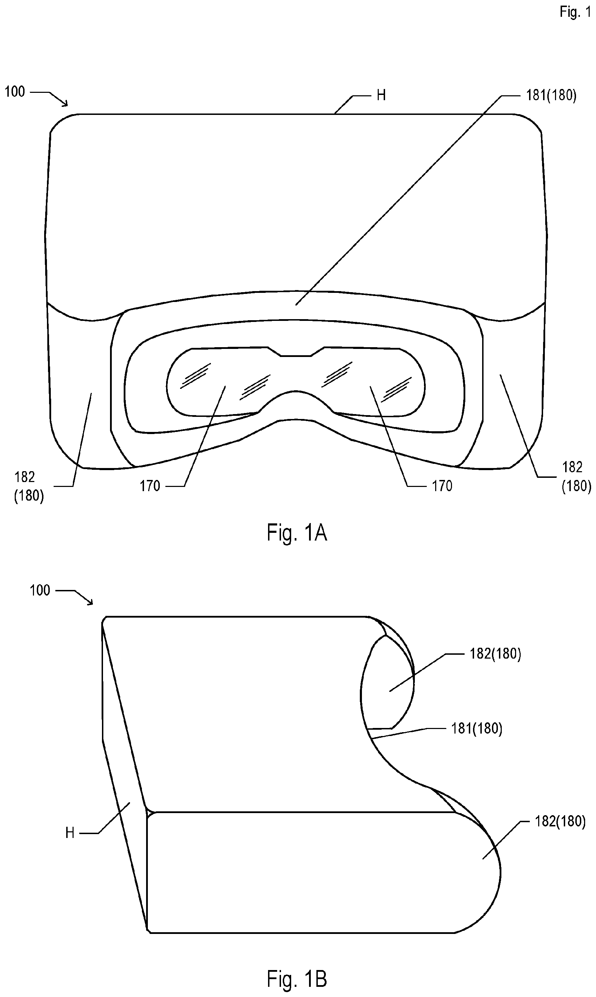

Figs. 1A and 1B are explanatory views illustrating an external appearance of an ophthalmic device 100 according to one embodiment of the present invention. The ophthalmic device 100 is assumed to be used, for example, in a site that is not a dark room such as a commercial facility, so that the subject can easily measure eye characteristics. As illustrated in the drawing, a light-shielding part 180 is provided in a housing H that houses optical components and the like of the ophthalmic device 100. The light-shielding part 180 includes a forehead contact part 181 and a side light-shielding part 182. A transparent window 170 is provided surrounded by the forehead contact part 181 and the side light-shielding part 182. The eye characteristics are measured in a state where the subject brings the forehead contact part 181 into contact with his/her forehead and looks into the window 170. When the subject brings the forehead into contact with the forehead contact part 181, light from above the subject is shielded. Further, the side light-shielding part 182 can shield the eye of the subject from a side of the face of the subject in a state where the forehead of the subject is in contact with the forehead contact part 181. Since both eyes are shielded from light by the forehead contact part 181 and the side light-shielding part 182, both eyes can be photographed in a dilated state (if even one eye is dazzled, both eyes will constrict). Further, a position of the eye of the subject can be roughly positioned by the forehead contact part 181 and the side light-shielding part 182.

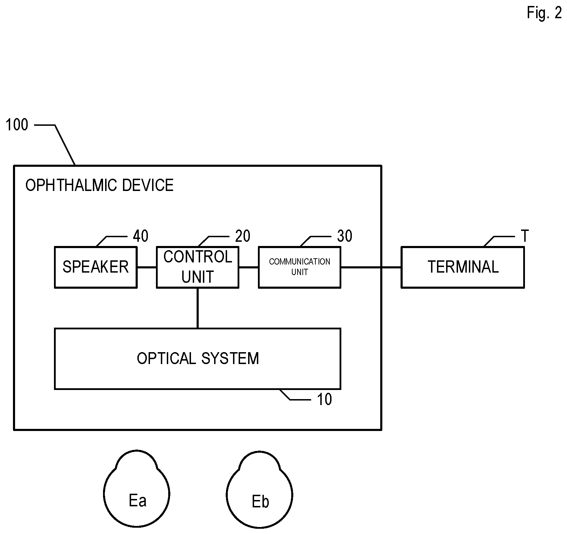

As illustrated in Fig. 2, an optical system 10 for measuring the eye characteristics of both eyes Ea and Eb, a control unit 20, a communication unit 30, and a speaker are disposed inside the housing H of the ophthalmic device 100. The control unit 20 includes a CPU, a ROM, a RAM, and a nonvolatile memory, and controls each unit of the ophthalmic device 100 by executing a control program recorded in the ROM or the nonvolatile memory by the CPU. The communication unit 30 is a communication interface for communicating with an external device in a wireless or wired manner. The control unit 20 can communicate with various terminals T via the communication unit 30. The terminal T is a device for inputting subject information, displaying an image captured by the ophthalmic device 100, and outputting measurement results of the eye characteristics, and the terminal T includes an input unit such as a keyboard, a touch panel, and a mouse, and a display (a display unit). The terminal T may be a portable terminal carried by the subject, or may be a PC, a tablet, or the like prepared in advance by an installer of the ophthalmic device 100. A printer or the like may be further connected to the terminal T. A speaker 40 outputs a guide voice or the like to the subject.

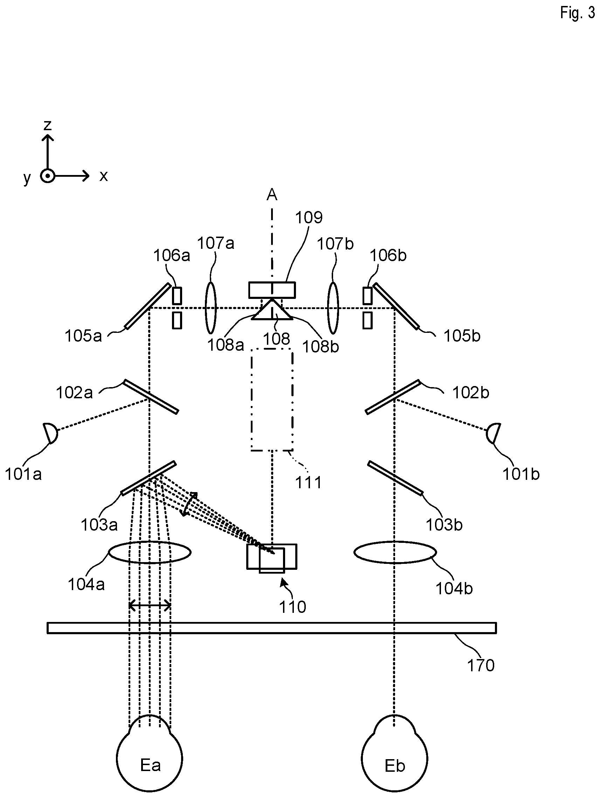

Fig. 3 is a diagram illustrating the configuration of the optical system 10 of the ophthalmic device 100 according to the first embodiment. The optical system 10 of the first embodiment includes an illumination optical system, an imaging optical system, and a measurement optical system. Reference numeral 109 denotes an image sensor. As illustrated in Fig. 3, a direction parallel to one side of a rectangular light receiving surface of the image sensor 109 is defined as an x axis, and a direction parallel to a side orthogonal to the one side of the light receiving surface is defined as a y axis. The light receiving surface of the image sensor 109 is parallel to an xy plane. A z axis is an axis perpendicular to the x axis and the y axis. The illumination optical system for illuminating each of the anterior eye portions of both eyes of the subject includes light sources 101a and 101b, half mirrors 102a and 102b, dichroic mirrors 103a and 103b, and objective lenses 104a and 104b. Ea represents the left eye of the subject, and Eb represents the right eye of the subject. The light sources 101a and 101b, the half mirrors 102a and 102b, the dichroic mirrors 103a and 103b, and the objective lenses 104a and 104b are arranged symmetrically with respect to a plane A that passes through the center of the light receiving surface of the image sensor 109 and is parallel to a yz plane.

The light emitted from the light sources 101a and 101b is reflected by the half mirrors 102a and 102b and transmitted through the dichroic mirrors 103a and 103b. The light transmitted through the dichroic mirrors 103a and 103b becomes parallel light by the objective lenses 104a and 104b and illuminates the eye to be examined.

A wavelength of the light emitted from the light sources 101a and 101b is about 800 nm, and a red point is visually recognized at a distance for the subject. The light sources 101a and 101b illuminate the entire anterior eye portions and form a virtual image at a corneal vertex of the eye to be examined. The position of the corneal vertex of the eye to be examined can be acquired by detecting this virtual image. Based on the position of the corneal vertex, guidance is provided to the subject so as to shift the position of the head of the subject relative to the housing H, or an angle of a movable mirror 110 described later is adjusted to scan measurement light.

The imaging optical system includes mirrors 105a and 105b, circular apertures 106a and 106b, imaging lenses 107a and 107b, a coupling mirror 108, and an image sensor 109. The mirrors 105a and 105b, the circular apertures 106a and 106b, the imaging lenses 107a and 107b, and the coupling mirror 108 are arranged symmetrically with respect to the plane A. Further, the coupling mirror 108 is a coupling body of the mirror 108a and the mirror 108b, and is disposed such that an intersection of the mirrors 108a and 108b is located on the plane A and is bilaterally symmetrical.

The light emitted from the light sources 101a and 101b is scattered at the anterior eye portion, and a part thereof is transmitted through the objective lenses 104a and 104b and further transmitted through the dichroic mirrors 103a and 103b. The light transmitted through the dichroic mirrors 103a and 103b is transmitted through the half mirrors 102a and 102b and reflected by the mirrors 105a and 105b.

The circular apertures 106a and 106b allow a part of the reflected light from the pair of mirrors 105a and 105b to pass therethrough. The light having passed through the circular apertures 106a and 106b is transmitted through the imaging lenses 107a and 107b, and forms an image on the mirrors 108a and 108b. The mirrors 108a and 108b constituting the coupling mirror 108 are disposed at an angle at which an incident light is reflected at a substantially right angle (right angle ± a predetermined angle) relative to optical axes of the pair of imaging lenses 107a and 107b. Note that, the predetermined angle includes 0 degrees, and may be a value larger than 0 degrees as long as vignetting described later falls within an allowable range. The image sensor 109 is disposed such that the light receiving surface is parallel to the optical axes of the imaging lenses 107a and 107b. In other words, the mirrors 108a and 108b are disposed such that the angle formed by them and the light receiving surface of the image sensor 109 is 45°.

In a case where the coupling mirror 108 is not disposed, the light transmitted through the imaging lenses 107a and 107b is designed such that an image of the anterior eye portion of each of both eyes is formed slightly behind the plane A (for example, 0.5 mm behind the plane A). The coupling mirror 108 is disposed such that an intersection portion of the mirrors 108a and 108b is close to the image sensor 109. Specifically, for example, the intersection portion is in contact with a protective glass of the image sensor 109, or the intersection portion is disposed close to the protective glass by about 0.2 mm. Since the intersection portion of the coupling mirror 108, which is located on the plane A, is close to the image sensor 109, it is possible to prevent the images of the anterior eye portions of the left and right eyes from being mixed in display areas of each other near a boundary between the binocular images where the light receiving surface of the image sensor 109 and the plane A intersect.

The pair of imaging lenses 107a and 107b are respectively disposed between a pair of circular apertures 106a and 106b and the coupling mirror 108. The circular apertures 106a and 106b are disposed at rear focal points of the objective lenses 104a and 104b (object-side telecentric). Therefore, even if the position of the eye to be examined is shifted in a front-back direction from the position assumed in advance (since a positional relationship between the forehead contact part 181 and the anterior eye portion can vary depending on the subject), the magnification of the image of the anterior eye portion is not changed and blurring occurs, so that a position detection error of the corneal vertex can be reduced. Further, the circular apertures 106a and 106b are disposed at front focal points of the imaging lenses 107a and 107b (image-side telecentric). Therefore, main light beams of the light transmitted through the imaging lenses 107a and 107b are parallel to the optical axes of the imaging lenses 107a and 107b and incident on the mirrors 108a and 108b, and are reflected at a right angle and incident on the image sensor 109.

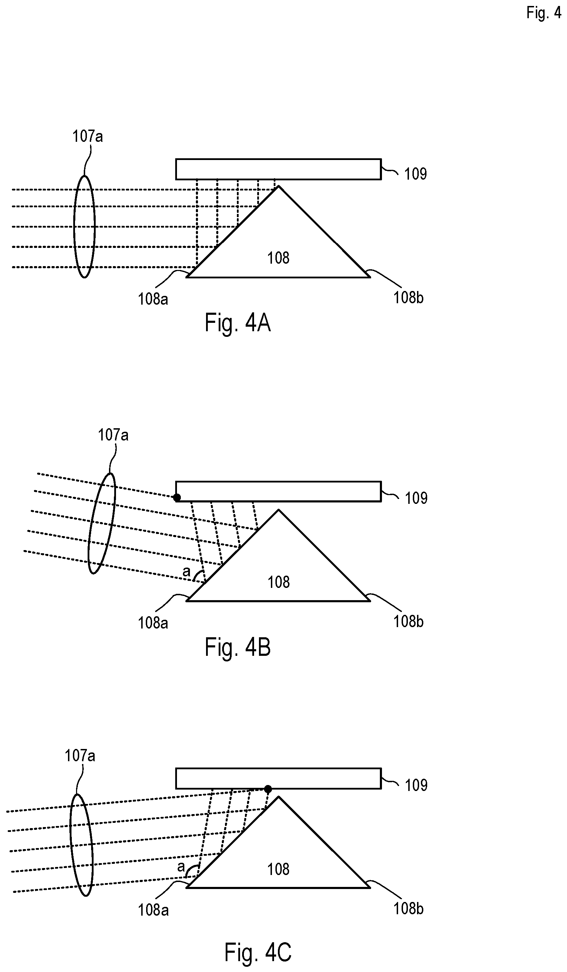

Fig. 4A is a diagram schematically illustrating that the light transmitted through the imaging lens 107a is incident on the mirror 108a and is reflected at a right angle. By making the imaging lenses 107a and 107b image-side telecentric, it is possible to suppress the images of the left and right eyes from being reflected on the other side beyond a boundary line (a line parallel to the y axis) between the left and right centers of the image sensor 109.

Note that, as illustrated in Fig. 4B, in a case where the imaging lens 107a is disposed such that an angle a formed by the incident light and the reflected light on the mirror 108a is an acute angle, the light in a peripheral portion is blocked by the image sensor 109, and thus, a shadow portion is generated (vignetting) in the image captured by the image sensor 109. Further, as illustrated in Fig. 4C, in a case where the imaging lens 107a is disposed such that the angle a of the mirror 108a is an obtuse angle, the light directly enters the image sensor 109 without being reflected by the mirror 108a, or a component reflected by the protective glass of the image sensor 109 and further reflected by the mirror 108a becomes noise and enters the image sensor 109. However, as in the present embodiment illustrated in Fig. 4A, the imaging lens 107a, the mirror 108a, and the image sensor 109 are disposed such that the reflected light is reflected at a right angle relative to the main light beam and is incident at a right angle relative to the light receiving surface of the image sensor 109, whereby occurrence of such a phenomenon (noise due to vignetting or stray light) can be prevented. Further, the present embodiment does not require an optical component as described in a second embodiment between the coupling mirror 108 and the image sensor 109, so that the cost can be suppressed.

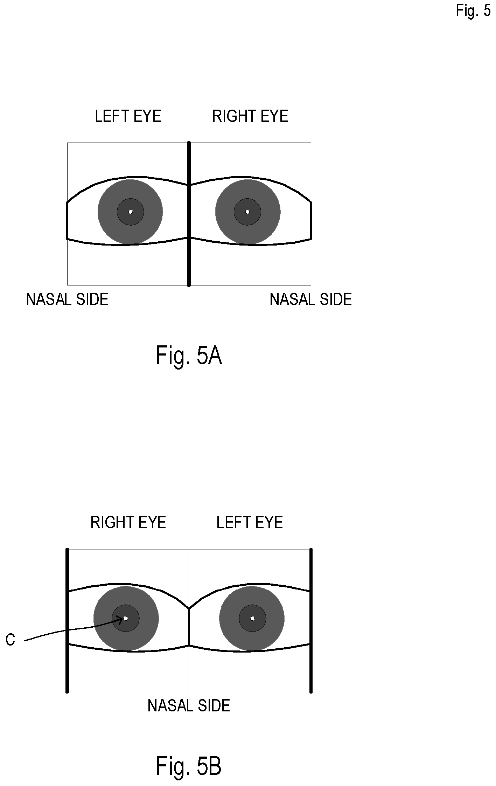

Fig. 5A illustrates an example of an image captured by the image sensor 109 in this manner. As illustrated in Fig. 5A, in the captured image, a left half is an image showing the left eye of the subject, and a right half is an image showing the right eye of the subject. Further, both left and right end portions in the entire captured image are images on a central portion side (a nasal side) of the face of the subject. For this reason, the control unit 20 performs image processing on the images of both eyes captured by the image sensor 109 so as to switch the left and right eyes, thereby generating an image as illustrated in Fig. 5B. In other words, in the image after the processing, the left half is an image showing the right eye, the right half is an image showing the left eye, and a central portion of the image is an image on the central portion side (the nasal side) of the face. Then, the control unit 20 outputs the image in which the left and right eyes have been switched to the terminal T via the communication unit 30. An image as illustrated in Fig. 5B can be displayed on the display unit of the terminal T. As a result, the subject or an examiner can easily intuitively identify the left eye and the right eye from this image, and can prevent a recognition error of the left and right images.

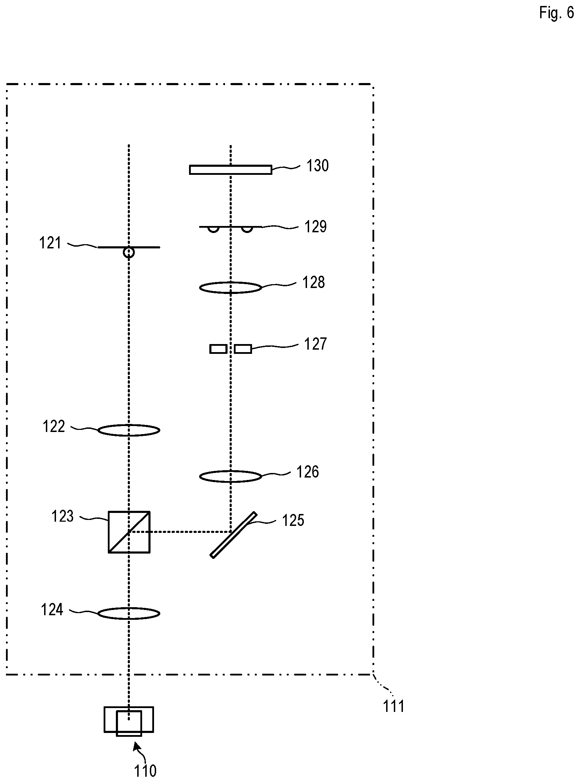

Fig. 6 is a diagram illustrating the configuration of an objective refractive power measurement optical system according to the present embodiment. The configuration of the measurement optical system will be described with reference to Figs. 3 and 6. Fig. 6 illustrates a specific example of an optical system 111 illustrated in Fig. 3 and the movable mirror 110. The measurement optical system includes a light source 121 that generates measurement light, a movable mirror 110 that selectively switches an irradiation direction of the measurement light to a direction of each of both eyes and irradiates both eyes with the measurement light, and a measurement unit that receives the measurement light reflected from both eyes. The optical component to which 122 to 130 is attached corresponds to the measurement unit.

The light emitted from the light source 121 becomes parallel light by a lens 122, is transmitted through a polarizing beam splitter 123, and is condensed on the movable mirror 110 by a lens 124. The light emitted from the light source 121 is adjusted in polarization so as to be P-polarized light relative to the polarizing beam splitter 123. As a result, almost 100% of the light emitted from the light source 121 is transmitted through the polarizing beam splitter 123.

The movable mirror 110 is configured, for example, by combining two uniaxial mirrors. Alternatively, the movable mirror 110 may be configured to drive one mirror in two axes. In any case, an irradiation position of the measurement light can be two-dimensionally controlled. The control unit 20 drives the movable mirror 110 by controlling a drive unit (not illustrated) such that the right eye Eb or the left eye Ea is alternatively irradiated with light at an angle. Fig. 3 illustrates an example in which the left eye Ea is irradiated with the measurement light. The light condensed on the movable mirror 110 is reflected so as to irradiate the left eye Ea, is reflected by the dichroic mirror 103a, is parallel to the optical axis of the objective lens 104a by the objective lens 104a, and is applied to the eye to be examined. The light irradiated to the eye to be examined is condensed and scattered near the eye fundus. In a case where the eye to be examined is an emmetropic eye, a part of the light scattered by the eye fundus is reflected by the objective lens 104a and the dichroic mirror 103a and condensed on the movable mirror 110. The condensed light becomes parallel light by the lens 124 (see Fig. 6), and only an S-polarized component is reflected by the polarizing beam splitter 123. The light reflected by the polarizing beam splitter 123 is reflected by a mirror 125 and condensed in an opening portion of a circular aperture 127 by the lens 126. The light having passed through the circular aperture 127 becomes parallel light by a lens 128, becomes a ring-shaped beam by a ring lens 129, and is projected on an image sensor 130. In a case where the eye to be examined is not an emmetropic eye, since an incident angle on the ring lens 129 is different from that of the emmetropic eye, a ring diameter imaged by the image sensor 130 is also different from that of the emmetropic eye (for example, the ring diameter becomes smaller in a case of a myopic eye and becomes larger in a case of a hyperopic eye). The control unit 20 acquires an ocular refractive power of the eye to be examined based on the ring diameter imaged by the image sensor 130.

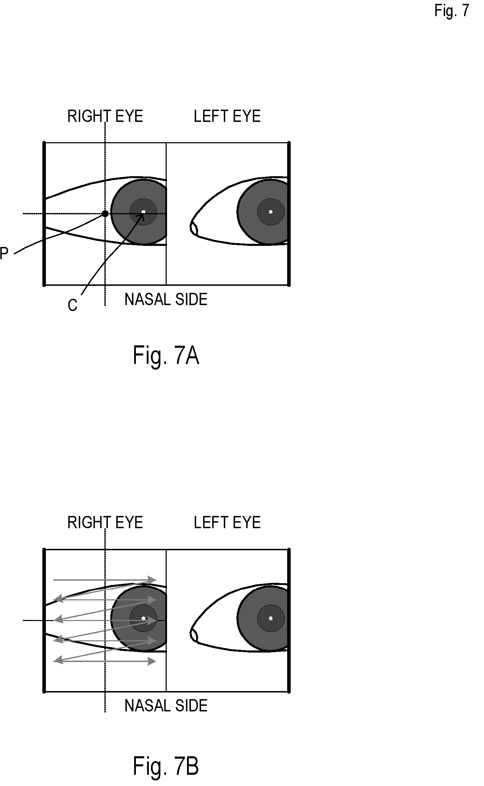

Further, the control unit 20 detects the position of a corneal vertex C (see Fig. 7A) in the image from the captured image and calculates the positional relationship with a reference point P in the image (an offset (the number of pixels) of a horizontal component of the image and an offset (the number of pixels) of a vertical component of the image). The control unit 20 specifies a driving amount of the movable mirror 110 according to the positional relationship, and drives the movable mirror 110. In this way, a scanning range of the measurement light is controlled such that the measurement light enters a predetermined range based on the corneal vertex C of the eye to be examined, and the ocular refractive power can be measured in a state in which the alignment can be performed. Trackability is higher than that in a case where eye alignment is performed by moving the light source 121 or the measurement unit, and examination accuracy can be improved. The predetermined range is determined in advance for each measurement target in the measurement optical system. For example, in a case where the ocular refractive power is measured, a circular range having a radius of a predetermined size centered on the corneal vertex C or a rectangular range including the circular range is determined as the predetermined range. The control unit 20 regards the position shifted from the reference point P by an offset amount indicating the positional relationship between the reference point P and the corneal vertex C, that is, the position of the corneal vertex C, as the center of the predetermined range. Then, the control unit 20 specifies the driving amount of the movable mirror 110 and drives the movable mirror 110 so that the eye to be examined is scanned over a predetermined range from the center. In a case where the offset amount exceeds a predetermined value, the control unit 20 outputs, from the speaker 40, a voice that prompts the subject to shift the position of the face. The voice message includes guidance of a moving direction of the face to reduce the offset amount.

Note that, as illustrated in Fig. 7B, the movable mirror 110 may be driven to scan the irradiation range of the measurement light up and down and left and right exhaustively at a predetermined pitch. In other words, the control unit 20 may drive the movable mirror 110 so that the light from the movable mirror 110 is emitted to a sufficiently wide range for measuring the measurement target in the measurement optical system. In this case, the control unit 20 extracts measurement data when the predetermined range with the corneal vertex as a reference is irradiated based on the above-described offset amount from the measurement results by the measurement optical system, and adopts the extracted measurement data. In the measurement of an axial length of the eye to be described later, the measurement data at a time point when the light coincides with the corneal vertex in a case where the irradiation is performed comprehensively as described above may be adopted.

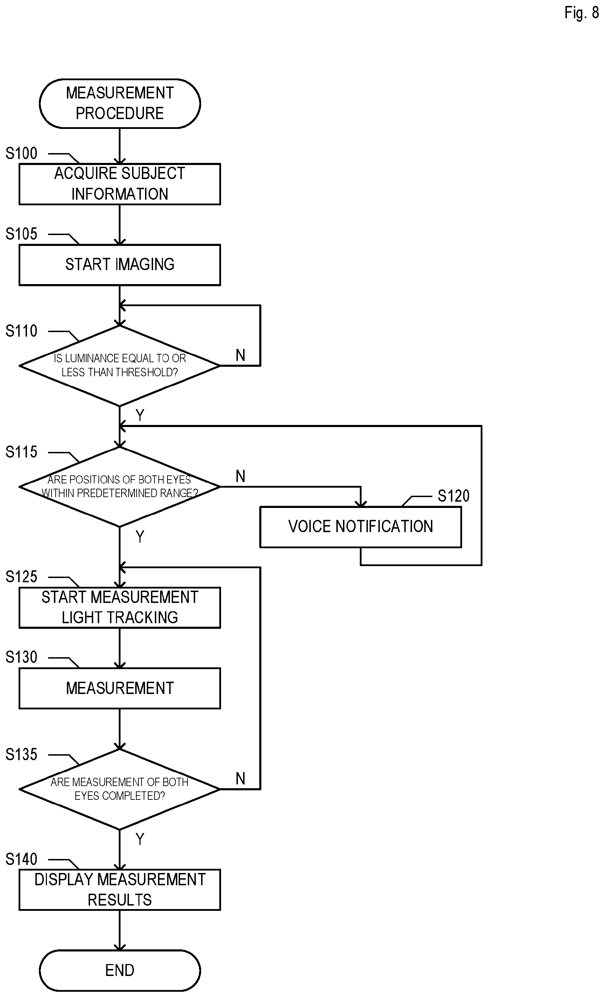

Fig. 8 is a flowchart illustrating measurement processing. The measurement processing is executed when a processing start trigger is detected. In the present embodiment, the processing start trigger is reception of the subject information from the terminal T. As the subject information, for example, information for identifying the subject (for example, name, patient ID, and the like), information indicating an attribute of the subject (for example, age group, sex, and the like), and the like are assumed. When the subject information is received from the terminal T via the communication unit 30, the control unit 20 acquires the subject information (step S100) and starts imaging (step S105). In other words, the control unit 20 causes the image sensor 109 to repeatedly capture images at a predetermined period.

When the subject brings the ophthalmic device 100 into contact with the face, a luminance of the image captured by the image sensor 109 is lower than that before the face comes into contact. By using this, the control unit 20 is configured to start measurement control of the eye characteristics in a case where the luminance of the image captured by the image sensor 109 becomes equal to or less than a threshold. Note that, as the luminance of the image, for example, a statistical value (mode value, average value, and the like) of the luminance of each pixel in the image captured by the image sensor 109 or the like is adopted. In step S110, the control unit 20 waits until the luminance becomes equal to or less than the threshold. In this way, by using the fact that the luminance is equal to or less than the threshold as a trigger for starting the measurement, it is not necessary for the subject to perform a specific operation for starting the measurement control, and it is possible to reduce an operator's workload. Note that, the "measurement control" includes tracking control of causing the measurement light to track such that the measurement light is emitted within a predetermined range based on the corneal vertex C, and control of measuring the eye characteristics after tracking is completed. In the present embodiment, the processing after step S115 is included in the "measurement control".

When it is determined in step S110 that the luminance is equal to or less than the threshold, that is, when it is estimated that the ophthalmic device 100 is in contact with the face of the subject, the control unit 20 determines whether or not the positions of both eyes are within a predetermined range (step S115). In other words, the control unit 20 determines for both eyes whether or not an absolute value of the offset amount between the reference point P and the corneal vertex C in the image is within a predetermined value.

When it is not determined in step S115 that the positions of both eyes are within the predetermined range, the control unit 20 performs audio notification (step S120). For example, the control unit 20 outputs, from the speaker 40, a message prompting to shift the position of the face relative to the ophthalmic device 100.

In a case where it is determined in step S115 that the positions of both eyes are within the predetermined range, the control unit 20 starts tracking of the measurement light (step S125) and performs the measurement (step S130). In other words, the control unit 20 drives the movable mirror 110 with the driving amount derived based on the offset amount of the corneal vertex C relative to the reference point P in the image captured by the image sensor 109, and irradiates the measurement light within a predetermined range based on the corneal vertex C to measure the ocular refractive power.

Subsequently, the control unit 20 determines whether or not the measurement of both eyes is completed (step S135). In a case where it is not determined that the measurement of both eyes has been completed, that is, in a case where only one eye has been completed, the control unit 20 returns to the processing of step S125 and performs measurement on the unmeasured eye. In other words, the control unit 20 drives the movable mirror 110 to emit the measurement light in the direction of the unmeasured eye, and performs the processing from step S125 onwards. In other words, according to the present embodiment, it is possible to measure the ocular refractive power of both eyes without moving the light source 121 or the measurement unit left or right. In a case where it is determined in step S135 that the measurement of both eyes has been completed, the control unit 20 displays the measurement results (step S140). In other words, the control unit 20 transmits the data indicating the measurement results and the captured image to the terminal T via the communication unit 30. The terminal T displays the measurement result data and the captured image received from the ophthalmic device 100 on the display unit.

As described above, according to the present embodiment, since both eyes can be photographed by one image sensor, the cost can be reduced as compared with a configuration in which each eye is photographed by another image sensor. Further, in the present embodiment, the subject can have both eyes photographed and measured by placing the forehead contact part 181 of the ophthalmic device 100 against his/her forehead. Therefore, it is not necessary to shift the ophthalmic device to left or right when measuring the right eye and the left eye, and the examination time can be shortened.

### (2) Second embodiment:

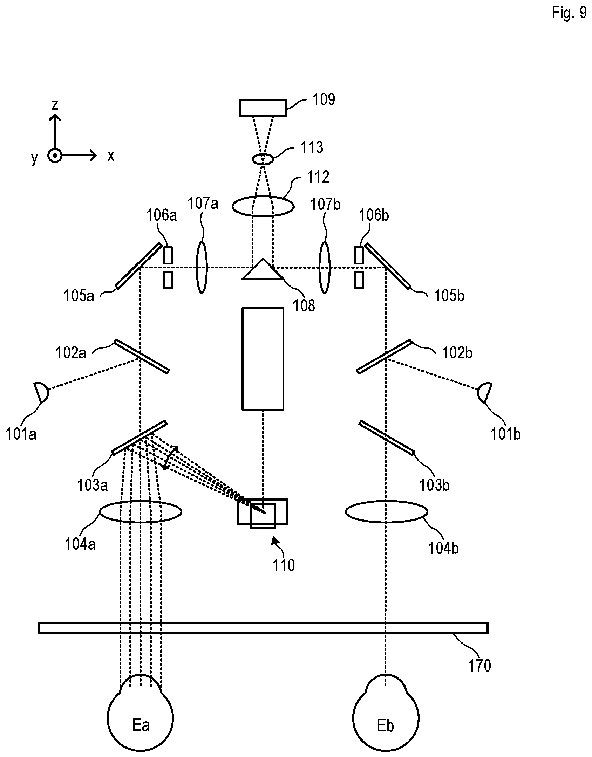

Fig. 9 is a diagram illustrating an optical system of an ophthalmic device according to a second embodiment. The second embodiment is different from the first embodiment in an imaging optical system. The imaging optical system in the second embodiment includes a relay lens 112 and an image sensor front lens 113 in addition to the configuration of the imaging optical system in the first embodiment. The other configurations common to the first embodiment are denoted by the same reference numerals, and the description thereof will be omitted. Ea represents the left eye of the eyes to be examined, and Eb represents the right eye.

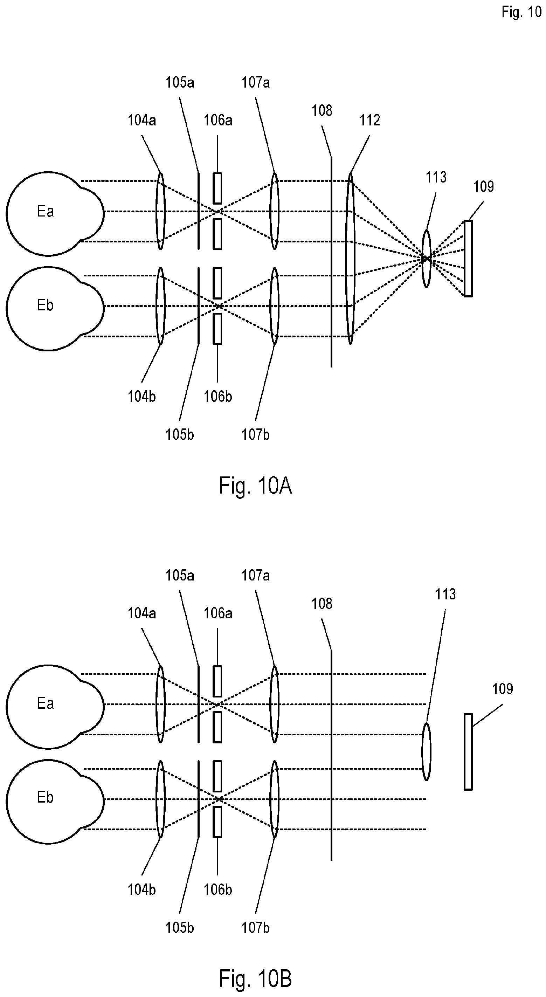

The image sensor front lens 113 is disposed between the coupling mirror 108 and the image sensor 109. The relay lens 112 is disposed between the coupling mirror 108 and the image sensor front lens 113. Fig. 10A is a diagram schematically illustrating the configuration of the imaging optical system according to the second embodiment, and is a diagram expressed such that optical axis directions changed by mirrors 105a and 105b and the coupling mirror 108 are aligned. As illustrated in the drawing, the image sensor front lens 113 is disposed at the rear focal point of the relay lens 112. A pair of circular apertures 106a and 106b is disposed so as to be conjugated with one image sensor front lens 113 relative to the imaging lenses 107a and 107b, the coupling mirror 108, and the relay lens 112. As a result, even when a lens diameter of the image sensor front lens 113 and the image sensor 109 are small, images of both eyes can be formed on one image sensor 109 without causing vignetting.

Note that, in a configuration in which a relay lens 12 is not provided in the configuration of the second embodiment, in a case where the image sensor front lens 113 is small as illustrated in Fig. 10B, the entire main light beam of the reflected light from both eyes Ea and Eb cannot be guided to the image sensor 109. However, the relay lens 12 can be omitted in the following cases.

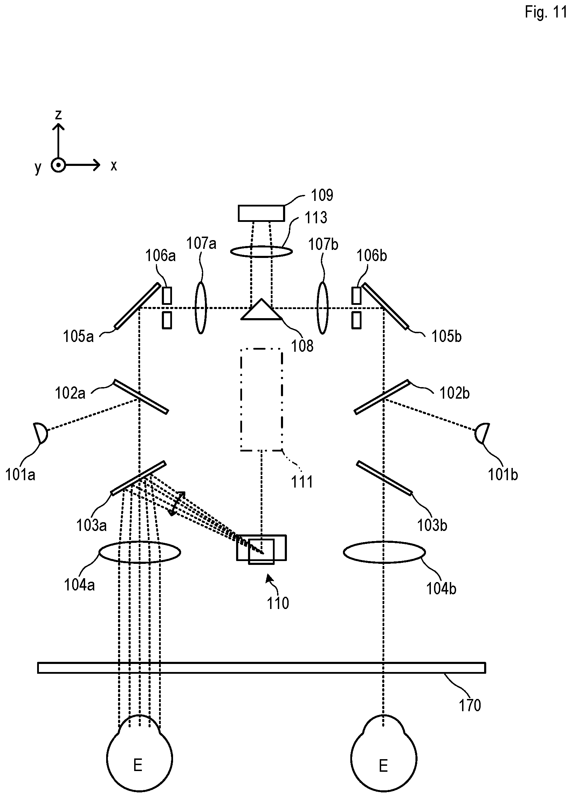

Fig. 11 illustrates a configuration example when the relay lens 112 is omitted in the second embodiment. In a case where the lens diameter of the image sensor front lens 113 is large enough to guide the entire main light beam of the reflected light from both eyes Ea and Eb to the image sensor 109, the relay lens 112 can be omitted. In this case, the image sensor 109 is disposed at a position closer to the image sensor front lens 113 than the focal point of the image sensor front lens 113, and the light reflected at a substantially right angle by the coupling mirror 108 is reduced by the image sensor front lens 113 and enters the image sensor 109.

### (3) Third embodiment:

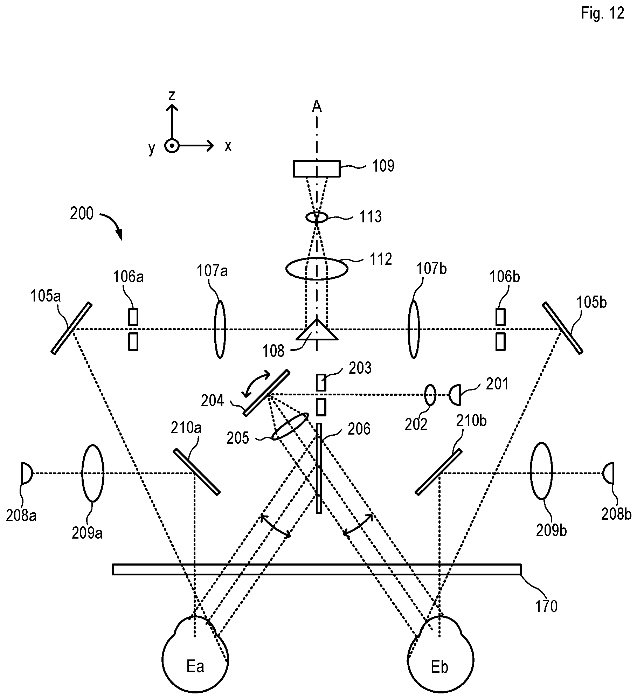

The configuration in which both eyes are imaged by one image sensor can also be applied to a slit lamp microscope. Fig. 12 is a diagram illustrating the configuration of an optical system of a slit lamp microscope 200 according to a third embodiment. The slit lamp microscope 200 includes an illumination optical system, a slit light generation optical system, a scanning optical system, and an imaging optical system. The illumination optical system for illuminating the anterior eye portions of both eyes Ea and Eb of the subject includes light sources 208a and 208b, lenses 209a and 209b, and total reflection mirrors 210a and 210b. The light sources 208a and 208b emit near-infrared light having a wavelength of approximately 800 nm. The light emitted from the light sources 208a and 208b arranged symmetrically becomes substantially parallel light by the lenses 209a and 209b, is reflected by the total reflection mirrors 210a and 210b, and is transmitted through the window 170 to illuminate the eye to be examined. Since the near-infrared light is made substantially parallel light, it is difficult for the subject to feel glare.

The slit light generation optical system for generating slit light includes a light source 201 that emits white light, a lens 202, and a slit-shaped opening portion 203. The light emitted from the light source 201 is narrowed in a long slit shape in a direction parallel to the y axis at the slit-shaped opening portion 203 through the lens 202.

The scanning optical system for scanning the slit light and irradiating the anterior eye portions of both eyes with the slit light (scanning the anterior eye portion by shifting the irradiation position of the slit light relative to the anterior eye portions at a predetermined interval) includes a scanning mirror 204, a lens 205, and a half mirror 206. The light narrowed in a slit shape by the slit-shaped opening portion 203 is reflected by the scanning mirror 204. The scanning mirror 204 is rotated by a drive mechanism (not illustrated) and scans the slit light within a predetermined range. The reflected light of the scanning mirror 204 is transmitted through the lens 205 to become substantially parallel light, and is split in the direction of the left and right eyes by the half mirror 206. The split slit light beam is projected onto the anterior eye portions of both eyes to be examined via the window 170. The half mirror 206 is disposed on the plane A and splits the slit light beam symmetrically. Therefore, according to this configuration, the slit light beam can be simultaneously projected to both eyes of the subject.

The imaging optical system includes the mirrors 105a and 105b, the circular apertures 106a and 106b, the imaging lenses 107a and 107b, the coupling mirror 108, the relay lens 112, the image sensor front lens 113, and the image sensor 109. The white slit light beam and the near-infrared illumination light irradiated to the eye to be examined are scattered at the anterior eye portions of the eye to be examined, and the scattered light is transmitted through the window 170 and reflected by the mirrors 105a and 105b. The light reflected by the mirrors 105a and 105b is guided to the image sensor 109 via the circular apertures 106a and 106b, the imaging lenses 107a and 107b, the coupling mirror 108, the relay lens 112, and the image sensor front lens 113, as in the second embodiment. In other words, the mirrors 105a and 105b are a pair of mirrors that guide the slit light reflected from the anterior eye portions of both eyes to the pair of coupling mirrors 108 (108a and 108b). Note that, also in the third embodiment, the relay lens 112 and the image sensor front lens 113 may be omitted, and the coupling mirror 108 and the image sensor 109 may be brought close to each other as in the first embodiment.

As described above, according to the present embodiment, both eyes can be photographed in a state where both eyes are simultaneously irradiated with the slit light. Further, since both eyes can be photographed at a time by one image sensor, it is not necessary for the subject to shift the ophthalmic device left or right in the right eye photographing and the left eye photographing, the examination time can be shortened, and a measurement data size can be halved as compared with a case of separately measuring the left and right eyes.

### (4) Other embodiments:

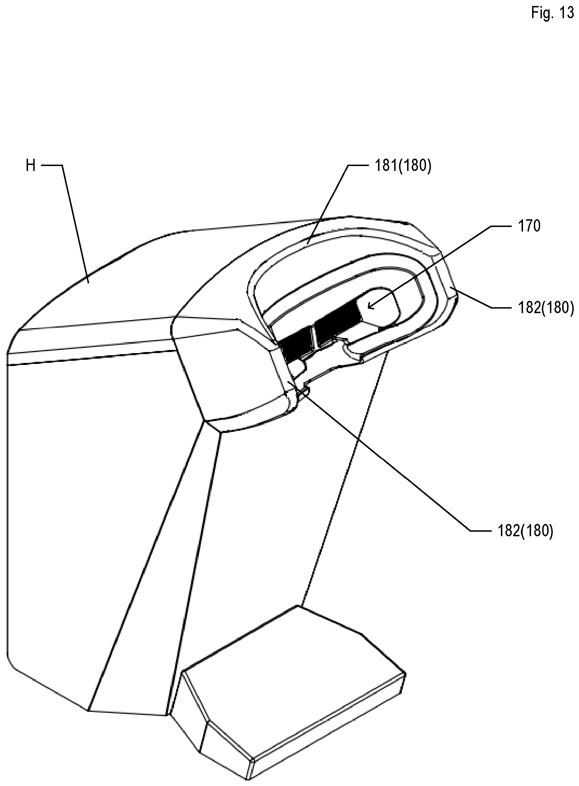

The above embodiment is an example for carrying out the present invention, and various other embodiments can be adopted. For example, the ophthalmic device may be a portable type as in the example illustrated in Fig. 1, or may be configured as a tabletop type that is attached or placed on a table such as a desk so that the subject can look into it, as illustrated in Fig. 13. Further, the light-shielding part may include a light-shielding part that shields light from below. For example, in the case of a portable type, a cheek contact portion that comes into contact with the cheek of the subject may be provided.

Further, the optical components constituting the imaging optical system, the illumination optical system, and the measurement optical system may be partially shared.

Further, for example, the ophthalmic device can be applied not only to the ocular refractive power measurement described in the first embodiment and the slit lamp microscope described in the third embodiment, but also to an optical interferometric axial length measurement, an optical coherence tomography device, a confocal scanning ophthalmoscope, and the like.

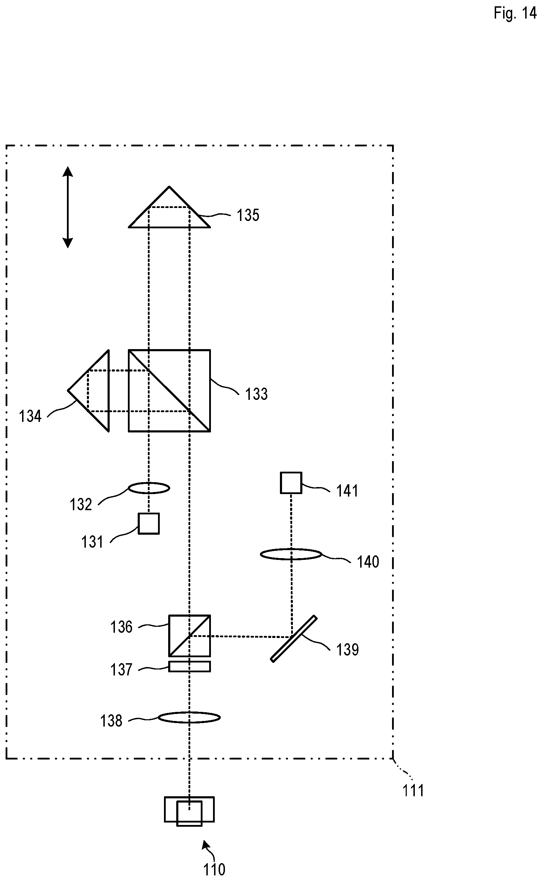

Fig. 14 is a diagram illustrating a configuration example of a measurement optical system for axial length measurement, and is a diagram illustrating a specific example of the optical system 111 in Fig. 3. The measurement optical system for axial length measurement includes a light source 131, a lens 132, a non-polarizing beam splitter 133, retroreflectors 134 and 135, a polarizing beam splitter 136, a λ/4 plate 137, a lens 138, a movable mirror 110, a mirror 139, a lens 140, and a detector 141. The light emitted from the light source 131 becomes parallel light by the lens 132 and is split into two directions by the non-polarizing beam splitter 133. The component reflected by the non-polarizing beam splitter 133 is reflected by the retroreflector 134 and is reflected by the non-polarizing beam splitter 133. The component transmitted through the non-polarizing beam splitter 133 is reflected by the retroreflector 135, transmitted through the non-polarizing beam splitter 133, and combined with the beam reflected by the retroreflector 134. There is an optical path length difference between an optical path of the light reflected by the retroreflector 134 and an optical path of the light reflected by the retroreflector 135. The retroreflector 135 is driven in the optical axis direction. The light combined by the non-polarizing beam splitter 133 becomes linearly polarized light having only P-polarized light by the polarizing beam splitter 136, and becomes circularly polarized light by the λ/4 plate 137. The beam circularly polarized by the λ/4 plate 137 is condensed on the movable mirror 110 by the lens 138. The light condensed by the movable mirror 110 is deflected so as to be applied to one of the left and right eyes, is reflected by the dichroic mirror 103a or 103b (see Fig. 3), becomes parallel light by the objective lens 104a or 104b, and is applied to the eye to be examined. The light applied to the eye to be examined is reflected by the cornea and the eye fundus. A part of the light scattered by the eye fundus is transmitted through the objective lens 104a or 104b, is reflected by the dichroic mirror 103a or 103b, and is condensed on the movable mirror 110. The condensed light becomes parallel light by the lens 138 (see Fig. 14) and becomes linearly polarized light having only the S-polarized component by the λ/4 plate 137, and is reflected by the polarizing beam splitter 136. The light reflected by the polarizing beam splitter 136 is reflected by the mirror 139, is transmitted through the lens 140, and reaches the detector 141 together with the reflected light from the cornea. When the optical path lengths of the light reflected by the cornea and the eye fundus match, an interference signal detected by the detector 141 becomes maximum. The control unit 20 acquires the eye axis length of the eye to be examined based on the position of the retroreflector 135 when the interference signal reaches its maximum.

Note that the ophthalmic device may be realized as an imaging device that images both eyes without the measurement optical system. The captured image may be output in the form of a still image or may be output in the form of a moving image.

The trigger for starting imaging in the image sensor 109 in the measurement processing is not limited to those described in the above embodiment. Imaging may be started in response to an instruction from the terminal T, or imaging may be started in a case where an operation unit of the ophthalmic device 100 is operated (for example, in a case where a power switch (not illustrated) is operated to turn on the power). In a case where the terminal T is configured to be able to associate the measurement results in the ophthalmic device with the subject information, the subject information does not need to be transmitted to the ophthalmic device. The subject information may be input either before or after the measurement.

Furthermore, the method of the present invention can also be applied as a program or a method. Further, the system, the program, and the method as described above may be implemented as a single device or may be implemented by using a component shared with another device, and include various aspects. Further, it can be changed as appropriate such as being partly software and partly hardware. Furthermore, the invention is also established as a recording medium of a program for controlling a system. Of course, the recording medium of the program may be a magnetic recording medium or a semiconductor memory, and can be considered in exactly the same manner in any recording medium to be developed in the future.

### REFERENCE SIGNS LIST

10 optical system,
12 relay lens,
20 control unit,
30 communication unit,
40 speaker,
100 ophthalmic device,
101a-101b light source,
102a-102b half mirror,
103a-103b dichroic mirror,
104a-104b objective lens,
105a-105b mirror,
106a-106b circular aperture,
107a-107b imaging lens,
108 coupling mirror,
108a-108b mirror (coupling mirror),
109 image sensor,
110 movable mirror,
112 relay lens,
113 image sensor front lens,
121 light source,
122 lens,
123 polarizing beam splitter,
124 lens,
125 mirror,
126 lens,
127 circular aperture,
128 lens,
129 ring lens,
130 image sensor,
131 light source,
132 lens,
133 non-polarizing beam splitter,
134 retroreflector,
134,135 retroreflector,
135 retroreflector,
136 polarizing beam splitter,
138 lens,
139 mirror,
140 lens,
141 detector,
170 window,
180 light-shielding part,
181 forehead contact part,
182 side light-shielding part,
200 slit lamp microscope,
201 light source,
202 lens,
203 slit-shaped opening portion,
204 scanning mirror,
205 lens,
206 half mirror,
208a-208b light source,
209a·209b lens,
210a-210b total reflection mirror,
A plane,
C corneal vertex,
H housing

## Claims

1. An ophthalmic device comprising:
an illumination optical system that illuminates each of anterior eye portions of both eyes to be examined;
an imaging optical system including a coupling mirror, a pair of mirrors that guide reflected light from each of the illuminated anterior eye portions of both eyes to a pair of the coupling mirrors, and one image sensor that images each of the anterior eye portions of both eyes based on the reflected light from the coupling mirror; and
a control unit that causes the image sensor to perform imaging.

2. The ophthalmic device according to claim 1, comprising:
a slit light generation optical system that generates slit light; and
a scanning optical system that scans the slit light and irradiates the anterior eye portions of both eyes with the slit light, wherein
the scanning optical system includes:
one scanning mirror that scans the slit light; and
a half mirror that splits the slit light reflected by the scanning mirror and guides the split slit light respectively to the anterior eye portions of both eyes, and
the imaging optical system includes
a pair of mirrors that guide the slit light reflected from each of the anterior eye portions of both eyes to a pair of the coupling mirrors.

3. The ophthalmic device according to claim 1 comprising a measurement optical system including:
a light source that generates measurement light;
a movable mirror that selectively switches an irradiation direction of the measurement light to a direction of each of the both eyes and irradiates the both eyes with the measurement light; and
a measurement unit that receives the measurement light reflected from the both eyes.

4. The ophthalmic device according to claim 3, wherein
the control unit rotates the movable mirror by a driving amount derived based on a positional relationship between a reference point and a corneal vertex in an image captured by the image sensor.

5. The ophthalmic device according to any one of claims 2 to 4, comprising a light-shielding part that shields the both eyes from light, wherein
the control unit starts measurement control of eye characteristics in a case where a luminance of an image captured by the image sensor becomes equal to or less than a threshold.

6. The ophthalmic device according to claim 1, wherein
the imaging optical system includes:
a pair of circular apertures through which a part of the reflected light from the pair of mirrors passes, respectively, and
a pair of imaging lenses respectively disposed between the pair of circular apertures and the coupling mirror, and
the circular aperture is disposed at a front focal point of the imaging lens,
the coupling mirror is disposed at an angle at which the coupling mirror reflects at a substantially right angle relative to optical axes of the pair of imaging lenses, and
the image sensor is disposed such that a light receiving surface is parallel to the optical axis of the imaging lens.

7. The ophthalmic device according to claim 6, wherein
the imaging optical system includes one image sensor front lens disposed between the coupling mirror and the image sensor.

8. The ophthalmic device according to claim 7, wherein
the imaging optical system includes a relay lens disposed between the coupling mirror and the image sensor front lens, and
the image sensor front lens is disposed at a rear focal point of the relay lens.

9. The ophthalmic device according to claim 1, wherein
the control unit performs image processing on images of the both eyes captured by the image sensor so as to switch left and right eyes, and causes a display unit to display an image in which the left and right eyes are switched.
